# EUROPEAN PATENT SPECIFICATION

(11) **EP 3 932 970 B1**
(45) Date of publication and mention of the grant of the patent: **26.08.2026**
(21) Application number: 20763054.2
(22) Date of filing: 19.02.2020
(51) Int. Cl.: C08G 59/50, C09J 11/04, C09J 11/06, C09J 163/02, C09J 163/04, A61B 1/00

(54) **ADHESIVE FOR ENDOSCOPE, CURED PRODUCT THEREOF, ENDOSCOPE, AND METHOD FOR MANUFACTURING THE SAME**
KLEBSTOFF FÜR ENDOSKOP, GEHÄRTETES PRODUKT DAVON SOWIE ENDOSKOP UND VERFAHREN ZU SEINER HERSTELLUNG
ADHÉSIF POUR ENDOSCOPE, PRODUIT DURCI ASSOCIÉ, ENDOSCOPE ET PROCÉDÉ DE FABRICATION ASSOCIÉ

(30) Priority: 26.02.2019 JP 2019032973
(43) Date of publication of application: 05.01.2022
(73) Proprietor: FUJIFILM Corporation, Tokyo 106-8620 (JP)
(72) Inventor: NAKAI, Yoshihiro, Ashigarakami-gun, Kanagawa 258-8577 (JP); FURUKAWA, Kazushi, Ashigarakami-gun, Kanagawa 258-8577 (JP)
(74) Representative: HGF
(86) International application number: PCT/JP2020/006542
(87) International publication number: WO 2020/175277

(56) References cited:
- WO-A1-2013/051458
- WO-A1-2015/093127
- WO-A1-2016/121795
- WO-A1-2019/036211
- JP-A- 2008 284 191
- JP-A- 2011 212 338
- JP-A- 2015 067 822
- US-A1- 2011 245 612
- US-A1- 2014 128 669
- US-A1- 2016 222 261

## Description

### 1. Field of the Invention

The present invention relates to adhesives for endoscopes, cured products thereof, endoscopes, and methods for manufacturing endoscopes.

### 2. Description of the Related Art

Endoscopes for observation of body cavities, the gastrointestinal tract, the esophagus, and other parts of a human body are repeatedly used. Therefore, the flexible tube that forms the insertion section of an endoscope is cleaned and disinfected with a chemical after each use. In particular, a cleanliness level that ensures sterilization beyond disinfection is required for insertion into a site where there is a high risk of infection, such as a bronchus. Accordingly, there is a need for an endoscope having high durability sufficient to withstand repeated high-level cleaning treatment.

The insertion section of an endoscope is inserted through the mouth or the nose into the body. It is desirable to reduce the diameter of the insertion section of an endoscope to alleviate discomfort and pain experienced by patients during insertion. Accordingly, adhesives are mainly used instead of bulky members such as screws to join members that form the insertion section.

Among adhesives, epoxy-based adhesives are frequently used to bond endoscope constituent members because these adhesives have high workability and cured products thereof have superior properties such as high adhesiveness, heat resistance, and moisture resistance.

For example, JP2017-214546A discloses an adhesive composition containing an epoxy resin as a main component and an inorganic both-ion exchanger. JP2017-214546A also discloses that, for example, the inorganic both-ion exchanger in the adhesive composition is composed of an inorganic compound containing a metal atom, and the adhesive composition may contain a curing agent such as xylenediamine, a polyamine, or a tertiary amine and may also contain an inorganic filler. According to JP2017-214546A, a cured product of the adhesive composition has high durability against sterilizing gas and is suitable for joining together endoscope constituent members.

JP2017-185086A discloses a thermosetting adhesive for medical devices that contains a thermosetting resin such as an epoxy resin and an infrared absorber dispersed in the thermosetting resin. According to JP2017-185086A, this adhesive can be quickly cured by heating with infrared radiation and can therefore alleviate the effect of thermal stress on medical devices.

JP2008-284191A discloses an adhesive for medical devices that contains a base adhesive containing at least one bisphenol epoxy resin selected from the group consisting of bisphenol A epoxy resins and bisphenol F epoxy resins as a base material in combination with a polyamide-amine-based curing agent. The base adhesive is mixed with multi-walled carbon nanotubes with diameters of 350 nm or less in an amount of 1 wt% to 30 wt%. According to JP2008-284191A, a cured product of the adhesive does not exhibit decreased adhesion strength when subjected to various disinfection processes and is also biocompatible.

JP2011-212338A discloses a flexible tube for endoscopes that includes an outer layer, an adhesiveness-enhancing layer formed around the outer layer and containing a soft epoxy resin, and an overcoat layer formed around the adhesiveness-enhancing layer and containing a fluorocarbon resin including vinylidene fluoride units. According to JP2011-212338A, when the flexible tube for endoscopes is subjected to autoclave sterilization treatment and hydrogen peroxide plasma sterilization treatment, damage and degradation to the outer layer can be inhibited, thus maintaining the required flexibility and protection.

US 2016/222261 A1 discloses a flexible tube for an endoscope, including: a metallic core material which has a helical tube formed by winding a band-shaped member in a helical form, and a reticulated tube formed along an outer periphery of the helical tube; a casing layer which is formed on a surface of the metallic core material, has a surface subjected to an atmospheric pressure plasma treatment, and contains an olefin-based elastomer or a styrene-based elastomer; an adhesiveness enhancing layer which is formed along an outer periphery of the casing layer and contains a soft epoxy resin; and an overcoat layer which is formed along an outer periphery of the adhesiveness enhancing layer and contains a fluororesin having a vinylidene fluoride unit.

WO 2019/036211 A1 discloses adhesive composition used in a medical instrument containing: a main agent which is an epoxy resin selected from the group consisting of bisphenol A-type epoxy resins, bisphenol F-type epoxy resins, and phenol novolac type epoxy resins; a curing agent comprising one or both of meta xylylene diamine and a derivative thereof; acrylic rubber, a filler which contains alumina.

US 2011/245612 A1 discloses an adhesive composition for medical devices of the present invention includes at least one epoxy resin (A) selected from the group consisting of a bisphenol A epoxy resin, a bisphenol F epoxy resin, and a phenol novolak epoxy resin, and modified silicone (B), wherein the epoxy resin (A) is mixed with the modified silicone B.

US 2014/128669 A1 discloses room temperature curing, two-component epoxy adhesives useful in bonding oily substrates include a resin side A and a curative side B. The resin side A includes an epoxy resin, a toughening component, a plasticizer and filler, and the curative side B includes a polyetheramine, amine-terminated butadiene rubber and a curing catalyst.

### SUMMARY OF THE INVENTION

The invention is defined by the appended claims.
Because endoscopes are repeatedly used over a long period of time, it is required that a state in which an endoscope member is secured with an adhesive can be sufficiently maintained after repeated use over a long period of time. In particular, the insertion section of an endoscope is exposed to high mechanical stress (e.g., vibrations and loads) due to repeated bending; therefore, it is required that the secured state can be sufficiently maintained after repeated exposure to such mechanical stress. As described above, it is also required that the secured state can be sufficiently maintained after repeated exposure to high-level cleaning treatment (sterilization treatment) involving chemical action, such as hydrogen peroxide plasma treatment. Unfortunately, after conducting research on epoxy-based adhesives in the related art, including those disclosed in the above patent documents, the inventors have found that these adhesives fail to simultaneously achieve the above mutually dissimilar types of durability required for endoscope applications at a sufficiently high level.

An object of the present invention is to provide an adhesive for endoscopes that is suitable for securing an endoscope constituent member and that can maintain sufficient adhesiveness after repeated exposure to mechanical stress or after repeated exposure to chemical sterilization treatment in a state in which the member is secured with the adhesive (in the form of a cured product), and also to provide a cured product of such an adhesive. Another object of the present invention is to provide an endoscope that exhibits less decrease in performance after repeated exposure to mechanical stress and chemical sterilization treatment as described above, and also to provide a method for manufacturing such an endoscope.

After conducting intensive research in view of the foregoing problem, the inventors have found that, if an epoxy-based adhesive contains an epoxy resin as a base material in combination with a particular polyamine compound as a curing component and further contains at least one of an inorganic filler, an amide compound, a fatty acid wax, or a sorbitol compound, a secured state in which a member is joined with the adhesive can be sufficiently maintained after repeated exposure to mechanical stress or after repeated exposure to sterilization treatment involving chemical action. The present invention has been made based on these findings and further research.

The foregoing objects of the present invention have been achieved by the invention as defined by the claims.

In the description of the present invention, a numerical range represented by "to" is meant to include values recited before and after "to" as lower and upper limits.

The adhesive for an endoscope according to the present invention can maintain sufficient adhesiveness after repeated exposure to mechanical stress or after repeated exposure to chemical sterilization treatment in a state in which an endoscope member is secured with the adhesive (in the form of a cured product). In addition, the cured product according to the present invention has high durability against repeated mechanical stress and also has high durability against repeated chemical sterilization treatment. In addition, the endoscope according to the present invention exhibits less decrease in performance after repeated exposure to mechanical stress or after repeated exposure to chemical sterilization treatment. Furthermore, the method for manufacturing an endoscope according to the present invention can provide an endoscope that exhibits less decrease in performance after repeated exposure to mechanical stress or after repeated exposure to chemical sterilization treatment.

### BRIEF DESCRIPTION OF THE DRAWINGS

FIG. 1 is an external view illustrating the configuration of an embodiment of an endoscope according to the present invention;
FIG. 2 is a partial sectional view illustrating the configuration of an insertion section of the endoscope illustrated in FIG. 1;
FIG. 3 is an external perspective view of a tip portion of the insertion section; and
FIG. 4 is a partially cutaway partial sectional view of the tip portion, in which hatching indicating cross-sections of lenses and a prism is omitted.

### DESCRIPTION OF THE PREFERRED EMBODIMENTS

### Adhesive for Endoscope

A preferred embodiment of an adhesive for an endoscope according to the present invention will now be described.

The adhesive for an endoscope according to the present invention (hereinafter also referred to as "adhesive according to the present invention") includes the following components (a) to (c):
(a) an epoxy resin including at least one of a bisphenol A epoxy resin, a bisphenol F epoxy resin, or a phenol novolac epoxy resin;
(b) a polyamine compound having an oxygen atom but no amide bond in the molecule thereof; and
(c) at least one of an inorganic filler, an amide compound, a fatty acid wax, or a sorbitol compound.

The epoxy resin (a) (hereinafter also simply referred to as "component (a)) is a base material for the adhesive. The polyamine compound (b) (hereinafter also simply referred to as "component (b)) is a curing component that reacts with the epoxy resin to cure the adhesive. The compound (c) (hereinafter also simply referred to as "component (c)) contributes to improved durability against mechanical stress and other factors, for example, by forming its own network different from the network of the epoxy resin in a cured product of the adhesive.

The form of the adhesive according to the present invention is not limited as long as it includes the above components. For example, the adhesive for an endoscope according to the present invention may contain a mixture of the components (a) to (c) (one-component type) or may include the components (a) to (c) in a state in which one of the components (a) to (c) is separated from the other components (two-component type). Alternatively, the adhesive for an endoscope according to the present invention may include the components (a) to (c) in a state in which the components (a) to (c) are separated from each other (three-component type). The adhesive according to the present invention encompasses all of these forms.

When the amounts of the components present in the adhesive are described in the present specification, or when the amounts of the components present in the adhesive are specified in the present invention, it is meant, in the case of a form such as a two-component type or a three-component type, that the components (a) to (c) are mixed together before use such that the individual components are present in the mixture in the desired amounts described or specified as above. That is, the individual components (a) to (c) do not have to be present in the amounts described in the present specification or specified in the present invention in a state in which the components are separated. In other words, in the case of a form such as a two-component type or a three-component type, it is meant that the components (a) to (c) are present in the amounts described in the present specification or specified in the present invention after the components (a) to (c) are mixed together before use.

If the adhesive for an endoscope according to the present invention is of a one-component type or is of a two-component or other type in which components that can react with each other have been mixed together (e.g., if the epoxy resin and the polyamine compound have been mixed together), the adhesive is preferably stored at a low temperature at which practically no reaction occurs in order to ensure that the components are stably maintained with no or sufficiently inhibited reaction with each other. For example, the adhesive can be stored at -20°C or lower, preferably -30°C or lower, more preferably -40°C or lower, even more preferably -50°C or lower. If necessary, light can be blocked during storage.

The adhesive according to the present invention may include, for example, solvents, plasticizers, adhesiveness enhancers (e.g., silane coupling agents), surfactants, colorants (e.g., pigments and dyes), weathering agents, antioxidants, heat stabilizers, lubricants, antistatic agents, whiteners, release agents, conductors, viscosity modifiers, fillers (e.g., silica and calcium carbonate), thixotropic agents, diluents, and flame retardants as long as they do not interfere with the advantages of the present invention.

A cured product obtained by curing the adhesive according to the present invention can maintain sufficient adhesiveness after repeated exposure to mechanical stress or after repeated exposure to chemical sterilization treatment in a state in which a member is secured with the adhesive. Although the mechanism is not fully understood, it can be attributed to, for example, the combined effect of the following factors: the component (b) has an oxygen atom but no amide bond in the molecule thereof and thus imparts moderate flexibility to the cured product so that it becomes tougher, and the component (c) forms its own network in the cured product.

The adhesive according to the present invention is suitable for securing various members that form endoscopes (endoscope constituent members). That is, the adhesive according to the present invention is suitable for use in bonding (joining) and securing an endoscope constituent member to another endoscope constituent member. The adhesive used for securing the endoscope constituent member becomes a cured product that forms a bonded region of the endoscope.

The member secured with the adhesive according to the present invention is not particularly limited. Examples of preferred members include metal members, glass members, and resin members. The endoscope constituent member is "secured" by bonding the endoscope constituent member to another member that forms the endoscope (support member). The support member may be a tube wall or other portion of the endoscope or an immovable member secured thereto, or may be a member, such as a tube, whose relative position can be changed within the endoscope. In the present invention, the term "secure" is meant to include filling, i.e., sealing, the space between the endoscope constituent member and the support member to which the member is to be joined with a cured product of the adhesive.

The individual components that form the adhesive according to the present invention will hereinafter be described.

### (a) Epoxy Resin

The adhesive according to the present invention includes an epoxy resin as the component (a). The epoxy resin includes at least one of a bisphenol A epoxy resin, a bisphenol F epoxy resin, or a phenol novolac epoxy resin. The adhesive according to the present invention may include one or more epoxy resins selected from the group consisting of bisphenol A epoxy resins, bisphenol F epoxy resins, and phenol novolac epoxy resins.

The proportion of the total amount of the bisphenol A epoxy resin, the bisphenol F epoxy resin, and the phenol novolac epoxy resin to the total amount of the epoxy resin present in the adhesive according to the present invention is preferably 70% by mass or more, more preferably 80% by mass or more, even more preferably 90% by mass or more. More preferably, the epoxy resin present in the adhesive according to the present invention is at least one of a bisphenol A epoxy resin, a bisphenol F epoxy resin, or a phenol novolac epoxy resin.

The epoxy equivalent weight of the epoxy resin present in the adhesive according to the present invention is preferably 10 to 1,000, more preferably 50 to 500, even more preferably 80 to 400, particularly preferably 100 to 300. The epoxy resin present in the adhesive according to the present invention typically has two or more epoxy groups per molecule.

The epoxy equivalent weight is determined by dividing the molecular weight of the epoxy compound by the number of moles of epoxy groups in the epoxy compound.

The bisphenol A epoxy resin that can be used in the adhesive according to the present invention is not particularly limited, and a wide range of bisphenol A epoxy resins commonly used as base materials for epoxy-based adhesives can be used. Specific examples of preferred bisphenol A epoxy resins include bisphenol A diglycidyl ethers (jER 825, jER 828, and jER 834 (all of which are trade names), manufactured by Mitsubishi Chemical Corporation) and bisphenol A propoxylate diglycidyl ethers (manufactured by Sigma-Aldrich Co.).

The bisphenol F epoxy resin that can be used in the adhesive according to the present invention is not particularly limited, and a wide range of bisphenol F epoxy resins commonly used as base materials for epoxy-based adhesives can be used. Specific examples of preferred bisphenol F epoxy resins include bisphenol F diglycidyl ethers (trade name: EPICLON 830, manufactured by DIC Corporation) and 4,4'-methylenebis(N,N-diglycidylaniline).

The phenol novolac epoxy resin that can be used in the adhesive according to the present invention is not particularly limited, and a wide range of phenol novolac epoxy resins commonly used as base materials for epoxy-based adhesives can be used. An example of such a phenol novolac epoxy resin is sold as Product No. 406775 from Sigma-Aldrich Co.

The amount of the epoxy resin present in the adhesive according to the present invention may be 5% to 90% by mass, more preferably 10% to 80% by mass. The amount of the epoxy resin present in the adhesive is also preferably 20% to 80% by mass, or preferably 30% to 80% by mass, or preferably 40% to 80% by mass, or preferably 50% to 75% by mass.

### (b) Polyamine Compound

The adhesive according to the present invention contains one or more polyamine compounds as the component (b). The polyamine compound serving as the component (b) has an oxygen atom in the molecule thereof. In addition, the polyamine compound serving as the component (b) has no amide bond (-NH-CO-) in the molecule thereof, which distinguishes the polyamine compound from polyamide-amines and also from the amide compound serving as the component (c). The polyamine compound serving as the component (b) is a compound having two or more amino groups having an active hydrogen per molecule. The polyamine compound preferably has an unsubstituted amino group (-NH₂), more preferably two or more unsubstituted amino groups. Even more preferably, the polyamine compound is a primary polyamine compound (i.e., a polyamine compound in which all amino groups are unsubstituted amino groups).

The polyamine compound serving as the component (b) preferably has 2 to 10, more preferably 2 to 8, even more preferably 2 to 6, still more preferably 2 to 4, particularly preferably 2 or 3, amino groups having an active hydrogen per molecule. In particular, at least one selected from the group consisting of diamine compounds and triamine compounds is suitable for use as the polyamine compound.

The active hydrogen equivalent weight (equivalent weight per active hydrogen in amino groups) of the polyamine compound serving as the component (b) is preferably 10 to 2,000, more preferably 20 to 1,000, even more preferably 30 to 900, still more preferably 40 to 800, still even more preferably 60 to 700, particularly preferably 65 to 600.

The active hydrogen equivalent weight is determined by dividing the molecular weight of the polyamine compound by the number of moles of active hydrogens in the amino groups of the polyamine compound (which means the molecular weight of the polyamine compound per active hydrogen in the amino groups).

The molecular weight of the polyamine compound serving as the component (b) is preferably 100 to 6,000, more preferably 100 to 3,000. If the polyamine compound is a polymer (e.g., if the polyamine compound has a polyoxyalkylene group, as described later), the molecular weight refers to number average molecular weight.

In particular, the polyamine compound serving as the component (b) preferably has an oxyalkylene structure, more preferably a polyoxyalkylene structure, in the molecule thereof to impart higher flexibility to the cured product so that it becomes tougher.

More preferably, the polyamine compound having an oxyalkylene structure is a polyoxyalkylenediamine compound or a polyoxyalkylenetriamine compound.

The alkylene group of the oxyalkylene structure may be a linear alkylene group or a branched alkylene group. The alkylene group of the oxyalkylene structure preferably has 1 to 10 carbon atoms, more preferably 2 to 6 carbon atoms, even more preferably 2 to 4 carbon atoms.

More preferably, the oxyalkylene structure is an oxyethylene structure or an oxypropylene structure.

If the polyamine compound serving as the component (b) has a polyoxyalkylene structure, the plurality of oxyalkylene groups that form the polyoxyalkylene structure may be the same as or different from each other. The average number of repeating units of oxyalkylene groups in the polyoxyalkylene structure is preferably 2 to 1,000, more preferably 3 to 500. The average number of repeating units is also preferably 2 to 100, or preferably 2 to 50, or preferably 2 to 35, or preferably 2 to 25. The polyamine compound serving as the component (b) may have a plurality of polyoxyalkylene structures.

Specific examples of preferred polyamine compounds that can be used in the present invention are given below. Numbers after parentheses are the average numbers of repeating units in the parentheses.

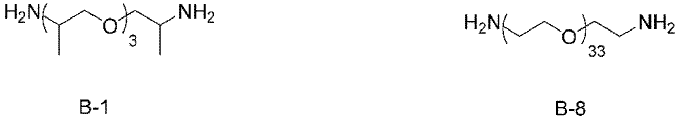

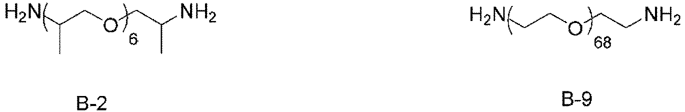

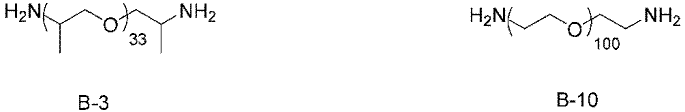

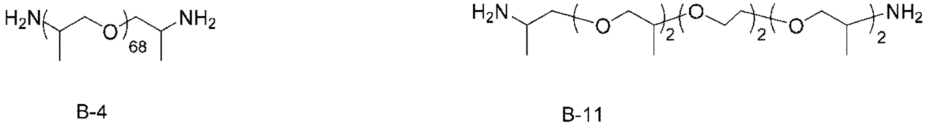

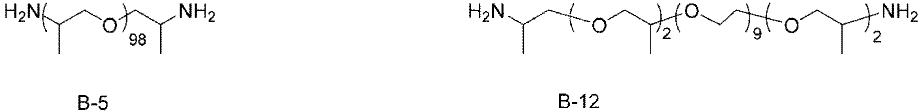

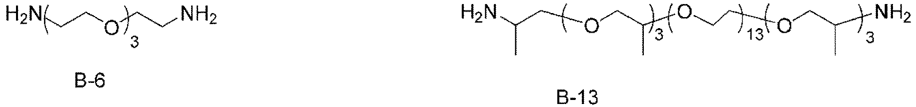

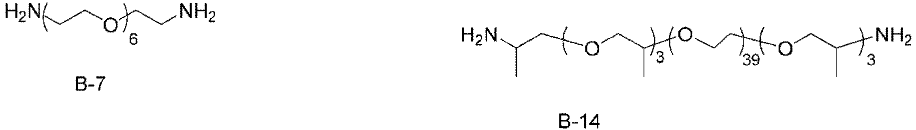

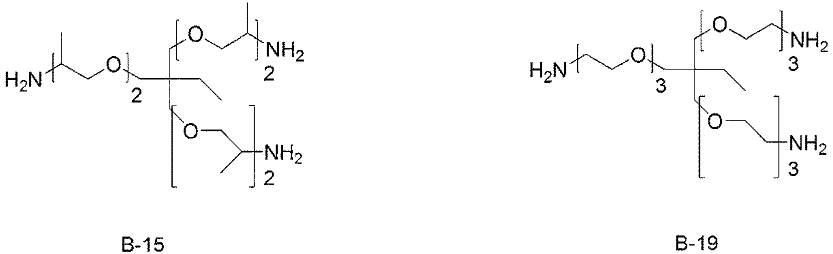

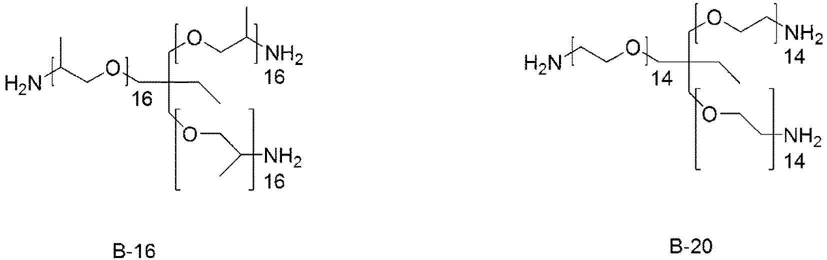

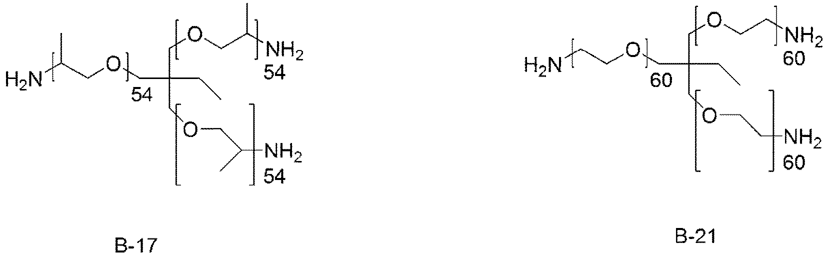

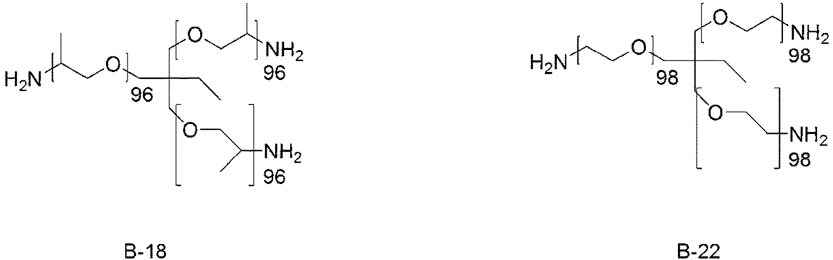

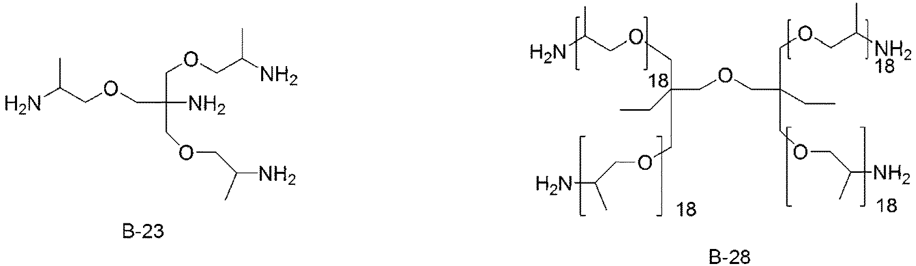

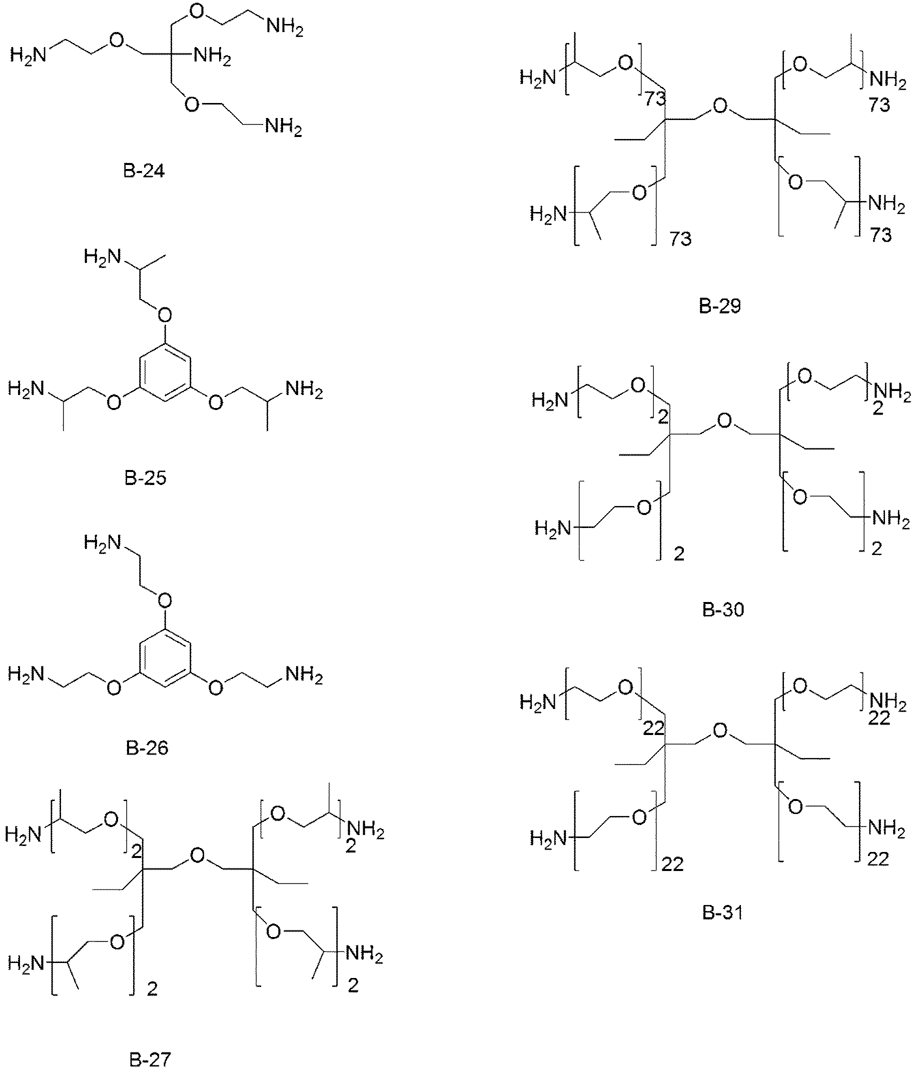

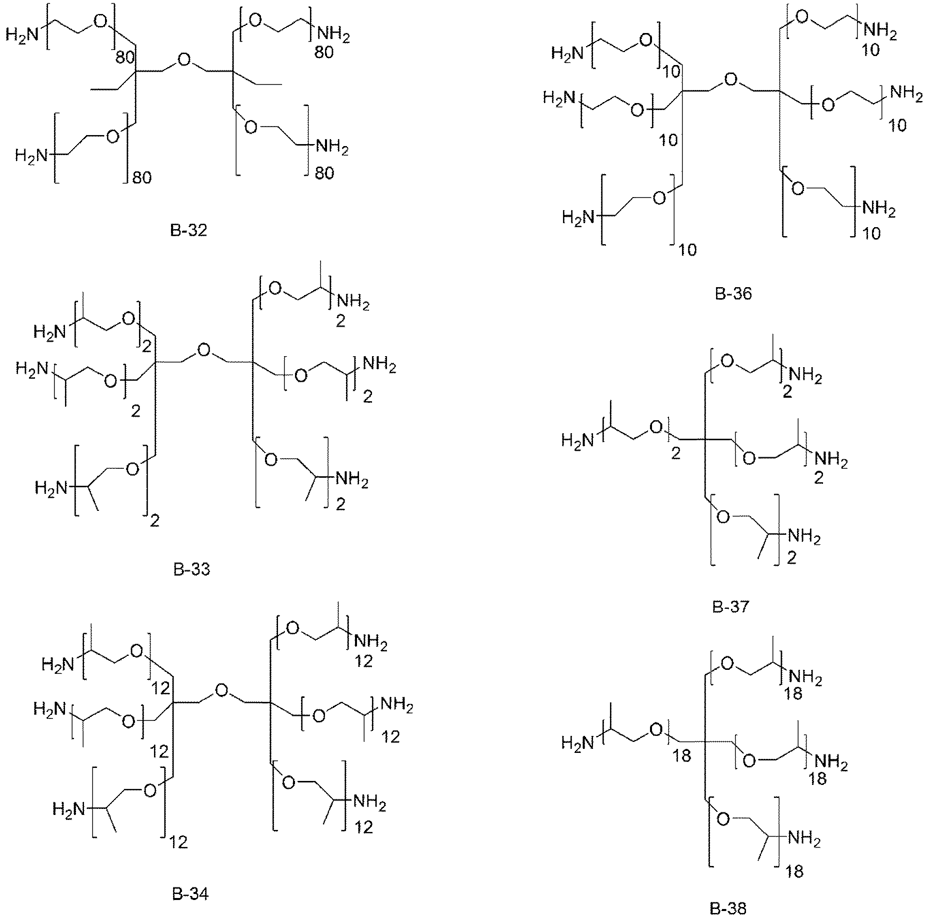

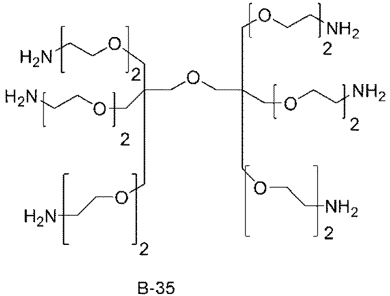

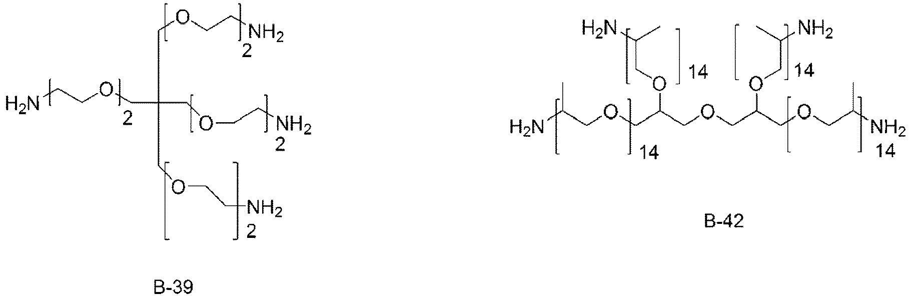

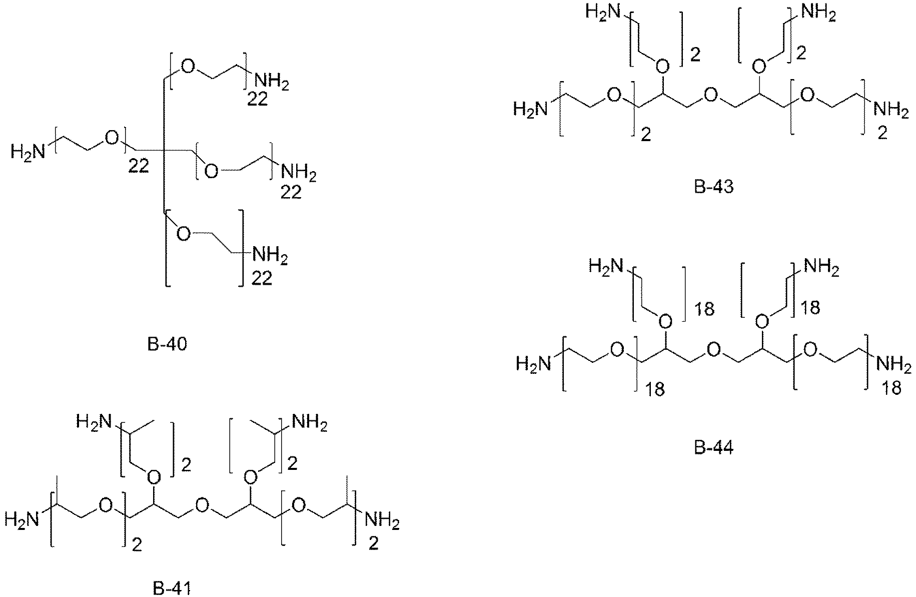

The polyamine compound serving as the component (b) can be synthesized as usual. Commercial products may also be used.

The amount of the polyamine compound serving as the component (b) present in the adhesive according to the present invention may be appropriately set by taking into account, for example, the active hydrogen equivalent weight and the molecular weight. For example, the amount of the polyamine compound serving as the component (b) may be 5 to 300 parts by mass, more preferably 10 to 250 parts by mass, even more preferably 15 to 220 parts by mass, based on 100 parts by mass of the epoxy resin. The amount of the polyamine compound serving as the component (b) is also preferably 5 to 200 parts by mass, or preferably 10 to 150 parts by mass, or preferably 10 to 100 parts by mass, or preferably 15 to 70 parts by mass, or preferably 15 to 50 parts by mass, based on 100 parts by mass of the epoxy resin. The ratio of the active hydrogen equivalent weight of the polyamine compound to the epoxy equivalent weight of the epoxy resin (active hydrogen equivalent weight/epoxy equivalent weight) is preferably 0.05 to 1.5, more preferably 0.05 to 1.2, even more preferably 0.06 to 1.0.

The adhesive according to the present invention may contain a curing component other than the polyamine compound serving as the component (b). The proportion of the polyamine compound serving as the component (b) to all curing component is preferably 80% by mass or more, more preferably 90% by mass or more. It is also preferred that all curing component be the polyamine compound serving as the component (b). If the adhesive according to the present invention contains a curing component other than the polyamine compound, various curing agents and curing aids known as curing components for epoxy-based adhesives can be used as the curing component. For example, the polyamine compound can be used in combination with at least one of an acid anhydride-based compound, an imidazole-based compound, a phosphorus-based compound, a thiol-based compound, a dicyandiamide-based compound, or a phenol-based compound.

### (c) Inorganic Filler, Amide Compound, Fatty Acid Wax, and Sorbitol Compound

The adhesive according to the present invention contains at least one of an inorganic filler, an amide compound, a fatty acid wax, or a sorbitol compound as the component (c). Inorganic Filler

Examples of inorganic fillers include so-called mineral fillers such as alumina (aluminum oxide), magnesia (magnesium oxide), titanium oxide (titanium white), aluminum hydroxide, barium titanate, zinc oxide, silica (including crystalline silica (silicon oxide) and fused silica (silicon oxide)), metal nanoparticles, and glass fiber; layered silicates such as talc, clay, mica, smectite, kaolin minerals, mica clay, and vermiculite; metal powders such as silver and copper powders; and nitrides such as aluminum nitride, boron nitride, silicon nitride, and gallium nitride. Other materials such as silicon carbide, carbon black, graphite, carbon fiber, and carbon nanotubes can also be used.

The inorganic filler is surface-treated according to the invention. The surface treatment is not particularly limited. For example, the inorganic filler may be surface-treated with a silane compound. In the present invention, "silane compound" refers to a compound having a structure in which at least one organic group is attached to Si, more preferably SiR⁴ (where R⁴ is an organic group). The silane compound is preferably a silane coupling agent, a silazane, or a silicone compound (polysiloxane). Such a surface treatment process can be performed as usual. For example, reference may be made to paragraphs [0090] to [0101] of JP2018-195964A.

The inorganic filler is commercially available. Examples of inorganic fillers are given below. Examples of commercial products of alumina include DAM-70, DAM-45, DAM-07, DAM-05, DAW-45, DAW-05, DAW-03, and ASFP-20 (all of which are trade names, manufactured by Denka Company Limited); AL-43-KT, AL-47-H, AL-47-1, AL-160SG-3, AL-43-BE, AS-30, AS-40, AS-50, AS-400, CB-P02, and CB-P05 (all of which are trade names, manufactured by Showa Denko K.K.); A31, A31B, A32, A33F, A41A, A43A, MM-22, MM-26, MM-P, MM-23B, LS-110F, LS-130, LS-210, LS-242C, LS-250, and AHP300 (all of which are trade names, manufactured by Nippon Light Metal Co., Ltd.); AA-03, AA-04, AA-05, AA-07, AA-2, AA-5, AA-10, and AA-18 (all of which are trade names, manufactured by Sumitomo Chemical Co., Ltd.); and AEROXIDE Alu C, AEROXIDE Alu C805, and AEROXIDE Alu 65 (all of which are trade names, manufactured by Nippon Aerosil Co., Ltd.).

Examples of commercial products of titanium oxide include G-1, G-10, F-2, F-4, and F-6 (all of which are trade names, manufactured by Showa Denko K.K.); TAF-520, TAF-500, TAF-1500, TM-1, TA-100C, and TA-100CT (all of which are trade names, manufactured by Fuji Titanium Industry Co., Ltd.); MT-01, MT-10EX, MT-05, MT-100S, MT-100TV, MT-100Z, MT-150EX, MT-100AQ, MT-100WP, MT-100SA, MT-100HD, MT-300HD, MT-500SA, MT-600SA, and MT-700HD (all of which are trade names, manufactured by Tayca Corporation); TTO-51(A), TTO-51(C), TTO-55(A), TTO-55(B), TTO-55(C), TTO-55(D), TTO-S-1, TTO-S-2, TTO-S-3, TTO-S-4, MPT-136, and TTO-V-3 (all of which are trade names, manufactured by Ishihara Sangyo Kaisha, Ltd.); and AEROXIDE TiO₂ NKT90 (trade name, manufactured by Nippon Aerosil Co., Ltd.).

Examples of commercial products of aluminum hydroxide include B-309 (trade name, manufactured by Tomoe Engineering Co., Ltd.); and BA173, BA103, B703, B1403, BF013, BE033, BX103, and BX043 (all of which are trade names, manufactured by Nippon Light Metal Co., Ltd.).

Examples of commercial products of layered silicates include NANO ACE D-1000, NANO ACE D-800, MICRO ACE SG-95, MICRO ACE P-8, and MICRO ACE P-6 (all of which are trade names for talc, manufactured by Nippon Talc Co., Ltd.); FH104, FH105, FL108, FG106, MG115, FH104S, and ML112S (all of which are trade names for talc, manufactured by Fuji Talc Industrial Co., Ltd.); Y-1800, TM-10, A-11, and SJ-005 (all of which are trade names for mica, manufactured by Yamaguchi Mica Co., Ltd.); and KUNIBIS-110 (trade name, manufactured by Kunimine Industries Co., Ltd.).

Examples of commercial products of barium titanate include BT-H9DX, HF-9, HF-37N, HF-90D, HF-120D, and HT-F (all of which are trade names, manufactured by KCM Corporation); the BT-100 and HPBT series (both of which are trade names, manufactured by Fuji Titanium Industry Co., Ltd.); the BT series (manufactured by Sakai Chemical Industry Co., Ltd.); and PALCERAM BT (manufactured by Nippon Chemical Industrial Co., Ltd.).

Examples of commercial products of zinc oxide include FINEX-30, FINEX-30W-LP2, FINEX-50, FINEX-50S-LP2, and XZ-100F (all of which are trade names, manufactured by Sakai Chemical Industry Co., Ltd.); FZO-50 (manufactured by Ishihara Sangyo Kaisha, Ltd.); and MZ-300, MZ-306X, MZY-505S, MZ-506X, and MZ-510HPSX (all of which are trade names, manufactured by Tayca Corporation).

Examples of commercial products of glass fiber include CS6SK-406, CS13C-897, CS3PC-455, and CS3LCP-256 (all of which are trade names, manufactured by Nitto Boseki Co., Ltd.); ECS03-615, ECS03-650, EFDE50-01, and EFDE50-31 (all of which are trade names, manufactured by Central Glass Co., Ltd.); and ACS6H-103 and ACS6S-750 (both of which are trade names, manufactured by Nippon Electric Glass Co., Ltd.).

Examples of commercial products of metal powders include AG3 and AG4, which are spherical silver powders, and FA5 and FA2, which are flaky silver powders (all of which are trade names, manufactured by DOWA Hightech Co., Ltd.); SPQ03R, SPN05N, SPN08S, and Q03R (all of which are trade names for silver powder, manufactured by Mitsui Mining & Smelting Co., Ltd.); AY-6010 and AY-6080 (both of which are trade names for silver powder, manufactured by Tanaka Kikinzoku Kogyo K.K.); ASP-100 (trade name for silver powder, manufactured by Aida Chemical Industries Co., Ltd.); the Ag-coated powder AG/SP (trade name for silver powder, manufactured by Mitsubishi Materials Electronic Chemicals Co., Ltd.); MA-O015K, MA-O02K, and MA-O025K (all of which are trade names for copper powder, manufactured by Mitsui Mining & Smelting Co., Ltd.); the electrolytic copper powders #52-C and #6 (manufactured by JX Nippon Mining & Metals Corporation); 10% Ag-coated Cu-HWQ (trade name for copper powder, manufactured by Fukuda Metal Foil & Powder Co., Ltd.); Type-A and Type-B (both of which are trade names for copper powder, manufactured by DOWA Electronics Materials Co., Ltd.); and UCP-030 (trade name for copper powder, manufactured by Sumitomo Metal Mining Co., Ltd.).

Examples of commercial products of nitrides include H grade, E grade, and H-T grade (all of which are trade names for aluminum nitride, manufactured by Tokuyama Corporation); TOYAL TecFiller TFS-A05P and TOYAL TecFiller TFZ-A02P (both of which are trade names for aluminum nitride, manufactured by Toyo Aluminium K.K.); ALN020BF, ALN050BF, ALN020AF, ALN050AF, and ALN020SF (all of which are trade names for aluminum nitride, manufactured by Tomoe Engineering Co., Ltd.); FAN-f05 and FAN-f30 (both of which are trade names for aluminum nitride, manufactured by Furukawa Denshi Co., Ltd.); Denka Boron Nitride SGP, Denka Boron Nitride MGP, Denka Boron Nitride GP, Denka Boron Nitride HGP, Denka Boron Nitride SP-2, and Denka Boron Nitride SGPS (all of which are trade names for boron nitride, manufactured by Denka Company Limited); UHP-S1, UHP-1K, UHP-2, and UHP-EX (all of which are trade names for boron nitride, manufactured by Showa Denko K.K.); and SN-9, SN-9S, SN-9FWS, SN-F1, and SN-F2 (all of which are trade names for silicon nitride, manufactured by Denka Company Limited).

Examples of commercial products of glass fiber include CF0027, CF0093, CF0018, and CF0033 (all of which are trade names, manufactured by Nippon Frit Co., Ltd.).

Examples of commercial products of silicon carbide include GMF-H type, GMF-H2 type, and GMF-LC type (all of which are trade names, manufactured by Pacific Rundum Co., Ltd.); and HSC1200, HSC1000, HSC059, HSC059I, and HSC007 (all of which are trade names, manufactured by Tomoe Engineering Co., Ltd.).

Examples of commercial products of silica include Silysia (manufactured by Fuji Silysia Chemical Ltd.); AEROSIL R972, AEROSIL R104, AEROSIL R202, AEROSIL 805, AEROSIL R812, AEROSIL RX200, AEROSIL R9200, AEROSIL 200, and AEROSIL R7200 (all of which are trade names, manufactured by Nippon Aerosil Co., Ltd.); the REOLOSIL series (manufactured by Tokuyama Corporation); CMC-12, VX-S, and VX-SR (all of which are trade names for crystalline silica, manufactured by Tatsumori Ltd.); FB-3SDC, FB-3SDX, SFP-30M, SFP-20M, SFP-30MHE, SFP-130MC, and UFP-30 (all of which are trade names for fused silica, manufactured by Denka Company Limited); and the EXCELICA series (trade name for fused silica, manufactured by Tokuyama Corporation).

Examples of commercial products of carbon fiber, carbon black, graphite, and carbon nanotubes include TORAYCA Milled Fiber MLD-30 and TORAYCA Milled Fiber MLD-300 (both of which are trade names for carbon fiber, manufactured by Toray Industries, Inc.); CFMP-30X and CFMP-150X (both of which are trade names for carbon fiber, manufactured by Nippon Polymer Sangyo Co., Ltd.); #1000 (trade name for carbon black, manufactured by Mitsubishi Chemical Corporation); XN-100 and HC-600 (both of which are trade names for graphite, manufactured by Nippon Graphite Fiber Co., Ltd.); SWeNT SG65, SWeNT SGi, IsoNanoTubes-M, IsoNanoTubes-S, PureTubes, Pyrograf PR-25-XT-PS, and PR-25XT-LHT (all of which are trade names for carbon nanotubes, manufactured by Sigma-Aldrich Co.); and MWNT MTC (trade name for carbon nanotubes, manufactured by Meijo Nano Carbon Co., Ltd.).

The inorganic filler used in the adhesive according to the present invention is preferably at least one of silica, titanium oxide, alumina, or a layered silicate. Such inorganic fillers are also surface-treated according to the invention. Inorganic fillers having at least an alkyl group introduced onto the surface thereof by surface treatment, that is, alkyl-modified inorganic fillers, may be used according to the invention. In particular, surface-treated silica (preferably alkyl-modified silica particles) is suitable.

The inorganic filler used in the present invention preferably has a volume average particle size of 1 to 10,000 nm, more preferably 3 to 5,000 nm, even more preferably 5 to 2,000 nm, still more preferably 5 to 1,000 nm, still even more preferably 6 to 500 nm, further preferably 7 to 200 nm, particularly preferably 8 to 100 nm. Silica, titanium oxide, and alumina preferably have a volume average particle size of 1 to 500 nm, more preferably 5 to 200 nm, even more preferably 6 to 100 nm, particularly preferably 7 to 50 nm.

The inorganic filler serving as the component (c) is preferably homogeneously dispersed in the adhesive after the components (a) to (c) are mixed together (before curing). Amide Compound

The amide compound is not particularly limited as long as it is a compound having an amide bond, preferably a carboxylic acid amide, more preferably a fatty acid amide (so-called amide wax) obtained by the reaction (condensation reaction) of a fatty acid with an amine.

The fatty acid is preferably a saturated or unsaturated fatty acid having 10 to 30 carbon atoms, more preferably 12 to 25 carbon atoms. Examples of such fatty acids include stearic acid, hydroxystearic acid, ricinoleic acid, palmitic acid, oleic acid, oxystearic acid, erucic acid, and lauric acid.

Examples of amines include alkylenediamines (e.g., alkylenediamines having 1 to 10 carbon atoms, specific examples of which include ethylenediamine and hexamethylenediamine), diethylenetriamines, triethylenetetramines, polyethylenepolyamines, ammonia, and alkylamines (e.g., alkylamines having 1 to 25 carbon atoms, specific examples of which include oleylamine and stearylamine).

In particular, reaction products of fatty acids with polyamines (preferably diamines, more preferably alkylenediamines) are suitable.

In the present invention, phrases such as "obtained by the reaction of X with Y" and "reaction product of X with Y" are meant to specify the structure of a product Z obtained by the reaction of X with Y. That is, even if X or Y is not used as a reactant, the reaction product of X with Y encompasses Z as long as the resulting product is Z.

Specific examples of preferred fatty acid amides include stearamide, oleamide, erucamide, behenamide, ethylene bisstearamide, hexamethylene bishydroxystearamide, N-oleyl palmitamide, and N-stearyl erucamide.

Examples of commercial products of amide compounds include NEUTRON, NEUTRON-2, NEUTRON-S, BNT-22H, SNT-F, and PNT-34 (all of which are trade names, manufactured by Nippon Fine Chemical Co., Ltd.); DISPARLON 6500 and DISPARLON 6900-20X (both of which are trade names, manufactured by Kusumoto Chemicals, Ltd.); and SLIPACKS E and SLIPACKS ZHH (trade names, manufactured by Mitsubishi Chemical Corporation).

The amide compound serving as the component (c) is preferably dissolved in the epoxy resin or solvent after the components (a) to (c) are mixed together (before curing). Fatty Acid Wax

The fatty acid wax is not particularly limited and may be, for example, a higher fatty acid, a higher alcohol, or an ester of a fatty acid with an alcohol (fatty acid ester compound).

As the fatty acid wax, waxes such as shellac wax, rice bran wax, insect wax, wool wax, and montan wax can be used. Also suitable are fatty acid waxes obtained by replacing some or all of the alcohol moieties of the above waxes (fatty acid esters) with other alcohols. Also suitable are fatty acid waxes obtained by converting the above waxes into metal salts of alkali metals such as sodium and potassium or alkaline earth metals such as calcium and magnesium.

The higher fatty acid may be a fatty acid having 8 to 24 carbon atoms, preferably 10 to 22 carbon atoms, more preferably 12 to 18 carbon atoms, even more preferably 16 to 18 carbon atoms. Specific examples of such higher fatty acids include caprylic acid, capric acid, lauric acid, myristic acid, palmitic acid, stearic acid, oleic acid, linoleic acid, linolenic acid, arachidic acid, behenic acid, 1-triacontanoic acid, and fatty acid mixtures such as coconut oil fatty acid, palm oil fatty acid, palm kernel oil fatty acid, and beef tallow fatty acid.

The higher alcohol may be a fatty alcohol having 8 to 60 carbon atoms. Specific examples of preferred higher alcohols include 2-dodecanol, 1-tetradecanol, 7-tetradecanol, 1-octadecanol, 1-eicosanol, 1,10-decanediol, and 1-triacontanol.

Esters of higher fatty acids with fatty alcohols (fatty acid ester compounds) are also preferred as the fatty acid wax.

Oils and fats may also be used as the fatty acid wax. Oils and fats are esters of glycerol with fatty acids, which are preferably higher fatty acids as mentioned above. Preferred oils and fats are those in which all three hydroxy groups of glycerol form esters with fatty acids (triglycerides). Oils and fats such as edible oils and fats are also suitable for use. Examples of edible oils and fats that can be used include soybean oil, linseed oil, rapeseed oil, tall oil, cottonseed oil, palm oil, coconut oil, castor oil, sunflower oil, corn oil, safflower oil, tung oil, canola oil, olive oil, sesame oil, rice bran oil, safflower oil, beef tallow, and fish oil. In particular, for example, soybean oil and linseed oil are suitable for use. It is also preferred to use fractionated oils, interesterified oils, and hardened oils derived from the above oils and fats, as well as mixtures thereof. In particular, hardened castor oil is preferred, and hydrogenated castor oil is especially suitable.

The fatty acid wax serving as the component (c) is preferably dissolved in the epoxy resin or solvent after the components (a) to (c) are mixed together (before curing).

### Sorbitol Compound

Examples of sorbitol compounds (compounds having a sorbitol skeleton) include dibenzylidene sorbitol compounds (compounds having a dibenzylidene sorbitol skeleton), tribenzylidene sorbitol compounds (compounds having a tribenzylidene sorbitol skeleton), and compounds derived therefrom. Among sorbitol compounds, a dibenzylidene sorbitol compound represented by formula (1) below is preferred since it can form a three-dimensional network structure by self-assembly at high temperature.

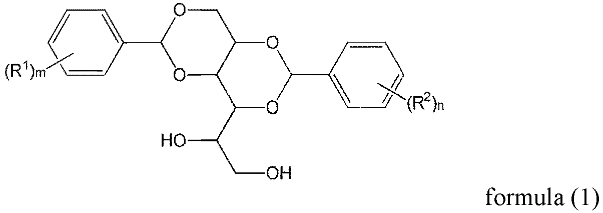

In formula (1), R¹ and R² represent an alkyl group having 1 to 5 carbon atoms, an alkoxy group having 1 to 5 carbon atoms, or a halogen atom. R¹ and R² may be the same as or different from each other. m and n are integers of 0 to 3.

The positions where R¹ and R² are attached to the benzene rings are not particularly limited.

The alkyl group having 1 to 5 carbon atoms that can be selected as R¹ and R² may be linear or branched. Examples of such alkyl groups include methyl, ethyl, isopropyl, n-butyl, and n-pentyl groups. The alkyl group having 1 to 5 carbon atoms that can be selected as R¹ and R² is preferably an alkyl group having 1 to 3 carbon atoms.

The alkoxy group having 1 to 5 carbon atoms that can be selected as R¹ and R² may be linear or branched. Examples of such alkoxy groups include methoxy, ethoxy, n-propoxy, isopropoxy, and n-butoxy groups. The alkoxy group having 1 to 5 carbon atoms that can be selected as R¹ and R² is preferably an alkoxy group having 1 to 3 carbon atoms.

Examples of halogen atoms that can be selected as R¹ and R² include fluorine, chlorine, bromine, and iodine atoms, preferably chlorine and bromine atoms.

Specific examples of preferred compounds represented by formula (1) above include 1,3:2,4-dibenzylidenesorbitol, 1,3:2,4-bis(p-methylbenzylidene)sorbitol, 1,3:2,4-bis(o-methylbenzylidene)sorbitol, 1,3:2,4-bis(p-ethylbenzylidene)sorbitol, 1,3:2,4-bis(p-chlorobenzylidene)sorbitol, 1,3:2,4-bis(p-methoxybenzylidene)sorbitol, 1,3:2,4-bis(2,4-dimethylbenzylidene)sorbitol, 1,3:2,4-bis(3,4-dimethylbenzylidene)sorbitol, and 1,3:2,4-bis(2,4,5-trimethylbenzylidene)sorbitol. Also preferred for use as the sorbitol compound in the present invention are 1,3-benzylidene-2,4-p-methylbenzylidenesorbitol, 1,3-p-methylbenzylidene-2,4-benzylidenesorbitol, 1,3-benzylidene-2,4-p-ethylbenzylidenesorbitol, 1,3-p-ethylbenzylidene-2,4-benzylidenesorbitol, 1,3-benzylidene-2,4-(3,4-dimethylbenzylidene)sorbitol, 1,3-(3,4-dimethylbenzylidene)-2,4-benzylidenesorbitol, 1,3-benzylidene-2,4-(2,4-dimethylbenzylidene)sorbitol, and 1,3-(2,4-dimethylbenzylidene)-2,4-benzylidenesorbitol.

The above compounds represented by formula (1) may be used alone or in a combination of two or more thereof. In particular, it is preferred to use a dibenzylidene sorbitol compound having identical substituted or unsubstituted benzylidene groups because it has good dispersibility and easily forms a three-dimensional network structure, and it is more preferred to use at least one of 1,3:2,4-dibenzylidenesorbitol, 1,3:2,4-bis(p-methylbenzylidene)sorbitol, 1,3:2,4-bis(o-methylbenzylidene)sorbitol, 1,3:2,4-bis(p-ethylbenzylidene)sorbitol, 1,3:2,4-bis(3,4-dimethylbenzylidene)sorbitol, or 1,3:2,4-bis(2,4,5-trimethylbenzylidene)sorbitol. Of the compounds mentioned above, it is particularly preferred to use at least one of 1,3:2,4-dibenzylidenesorbitol or 1,3:2,4-bis(p-methylbenzylidene)sorbitol.

The sorbitol compound serving as the component (c) is preferably dissolved in the epoxy resin or solvent after the components (a) to (c) are mixed together (before curing).

In particular, the adhesive according to the present invention preferably contains an inorganic filler, more preferably surface-treated silica particles, as the component (c).

In the present invention, the component (c) is believed to form a network independent of the network of the epoxy resin serving as the base material in the adhesive or the cured product thereof by relatively weak bonding (cohesive force), and the formation of its own network will effectively improve durability against mechanical stress.

In the present invention, the above components (c) may be used alone or in a combination of two or more thereof.

The amount of the component (c) present in the adhesive according to the present invention may be appropriately adjusted depending on the purpose. For example, the amount of the component (c) present in the adhesive may be 1 to 50 parts by mass, more preferably 2 to 40 parts by mass, even more preferably 5 to 30 parts by mass, still more preferably 8 to 25 parts by mass, particularly preferably 12 to 20 parts by mass, based on 100 parts by mass of the epoxy resin.

### Cured Product

A cured product according to the present invention is a cured product formed by curing the adhesive according to the present invention. Specifically, the cured product according to the present invention is used as a member that forms a bonded region of an endoscope. The curing temperature of the adhesive according to the present invention is not particularly limited and is appropriately adjusted depending on the purpose by taking into account, for example, the heat resistance of the member to be bonded and the curing time. It is preferred to mix the individual components together while removing bubbles. To this end, mixing is typically performed under reduced pressure. The curing temperature is preferably 100°C or lower, more preferably 80°C or lower, even more preferably 60°C or lower, or may be 50°C or lower. To sufficiently perform the curing reaction, the curing temperature is preferably 0°C or higher, more preferably 10°C or higher. The curing reaction time can be appropriately set depending on the purpose. Typically, the curing reaction is performed for 1.5 to 200 hours to obtain a cured product.

### Endoscope

An endoscope according to the present invention includes a constituent member secured with a cured product according to the present invention. "Constituent member secured with a cured product according to the present invention" means that at least one of the members that form the endoscope is secured to a support member with a cured product according to the present invention therebetween.

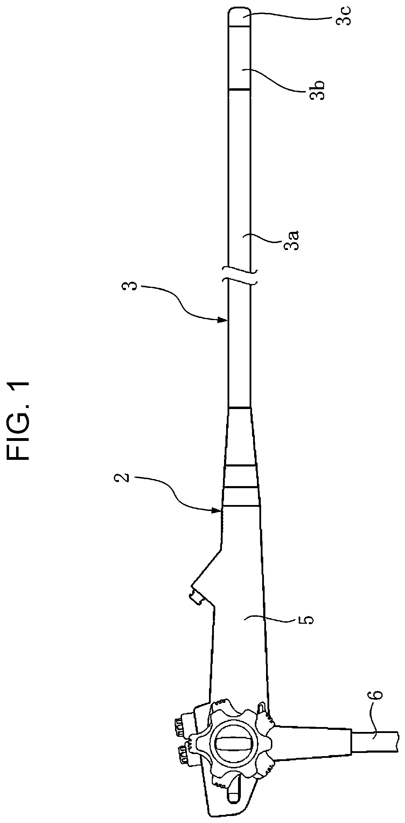

An example of an endoscope (electronic endoscope) according to the present invention will now be described. Electronic endoscopes, which are widely used as medical devices, incorporate a flexible tube for an endoscope (a flexible tube for an endoscope may be hereinafter simply referred to as "flexible tube"). In the example illustrated in FIG. 1, an electronic endoscope 2 is composed of an insertion section 3 for insertion into a body cavity, a main-body operating section 5 connected to the proximal end portion of the insertion section 3, and a universal cord 6 for connection to a processor device and a light source device. The insertion section 3 is composed of a flexible tube 3a connected to the main-body operating section 5, an angle portion 3b connected to the flexible tube 3a, and a tip portion 3c connected to the distal end of the angle portion 3b and composed mainly of a metal (e.g., stainless steel) member. The tip portion 3c has an imaging device (not illustrated) built thereinto for imaging the interior of a body cavity. The flexible tube 3a, which accounts for most of the length of the insertion section 3, is flexible substantially over the entire length thereof. In particular, the portion to be inserted into a site such as a body cavity has a more flexible structure.

In FIG. 1, a plurality of channels (tubes, not illustrated) are formed so as to extend axially through the insertion section 3 from the main-body operating section 5 to the distal end face of the tip portion 3c.

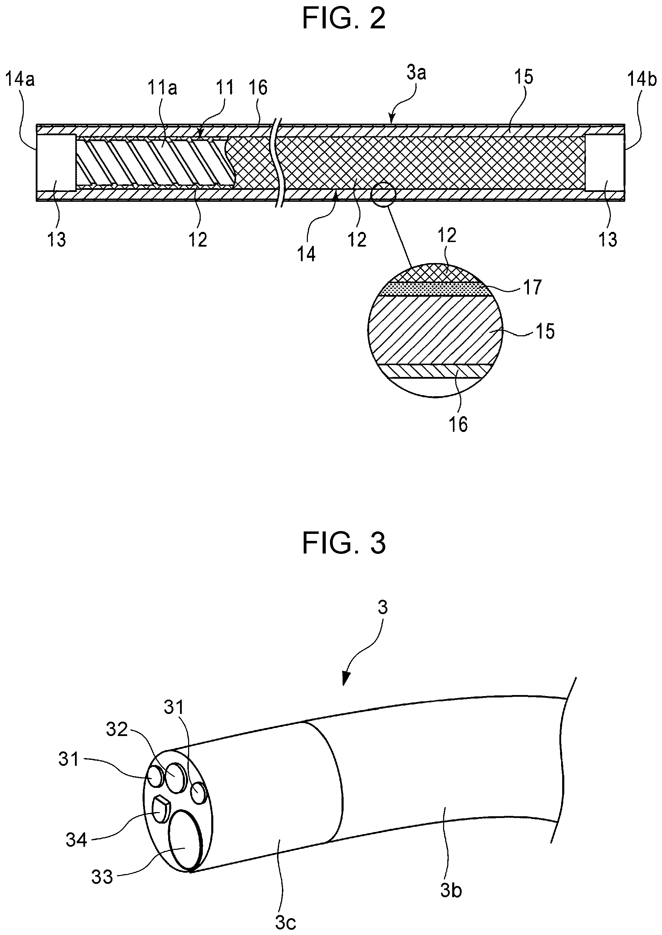

As illustrated in FIG. 2, the flexible tube 3a in FIG. 1 is composed of a flexible tube substrate 14 and a resin layer 15 covering the outer peripheral surface of the flexible tube substrate 14.

Reference numeral 14a denotes the distal side (tip portion 3c side), whereas reference numeral 14b denotes the proximal side (main-body operating section 5 side).

The flexible tube substrate 14 includes a spiral tube 11 disposed on the innermost side and formed by spirally winding a metal strip 11a and a tubular net 12 covering the spiral tube 11 and formed by weaving metal wires. Caps 13 are fitted to both ends of the flexible tube substrate 14. The resin layer 15 is bonded to the flexible tube substrate 14 with an adhesive cured product layer 17 therebetween. The adhesive cured product layer 17 can be formed by applying and curing the adhesive according to the present invention. Although the adhesive cured product layer (bonded region) 17 is illustrated as a layer with uniform thickness for illustration purposes, it does not necessarily have to be in that form, but may be present in irregular form between the resin layer 15 and the flexible tube substrate 14. Rather, the adhesive cured product layer 17 may have negligible thickness, and the resin layer 15 and the flexible tube substrate 14 may be bonded together substantially in contact with each other.

The outer surface of the resin layer 15 is coated with a chemical-resistant coat layer 16 such as one containing fluorine. To clearly illustrate the layer structure, the adhesive cured product layer 17, the resin layer 15, and the coat layer 16 are shown as being thick relative to the diameter of the flexible tube substrate 14.

As illustrated in FIG. 3, illumination windows 31, an observation window 32, and a forceps port 33 are formed in the distal end face of the tip portion 3c. A nozzle 34 for ejecting water and air is also formed in order to clean the distal end face where necessary. The illumination windows 31, the observation window 32, the forceps port 33, and the nozzle 34 are connected to the main-body operating section 5 through the channels.

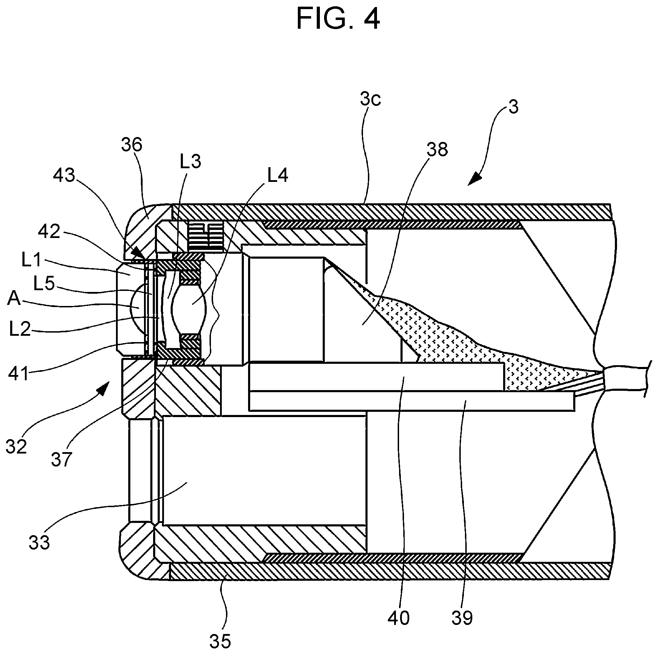

As illustrated in FIG. 4, the tip portion 3c is composed of a tip-portion main body 35 formed of a metal and a distal end cap 36 formed of an electrically insulating material.

An observation unit 43 is an optical system device installed in the observation window 32. The observation unit 43 includes an objective optical system composed of lenses L1 to L5 secured within a lens holder 37 with adhesive cured products 41 and 42. The adhesive cured products 41 and 42 can be formed by applying and curing the adhesive according to the present invention. In the objective optical system, reference character A denotes an air layer. A prism 38 is bonded and secured to the end face of the lens holder 37. The prism 38 bends the optical axis of the objective optical system at a right angle. The prism 38 is secured to a solid-state imaging element 40. The solid-state imaging element 40 is secured to a substrate 39. These members can also be secured by applying the adhesive according to the present invention.

### Method for Manufacturing Endoscope

A method for manufacturing an endoscope according to the present invention is not particularly limited as long as the method includes securing an endoscope constituent member with the adhesive according to the present invention. As for the steps other than securing the endoscope constituent member, common manufacturing steps can be employed to manufacture the endoscope according to the present invention.

The material of the endoscope constituent member to be secured is not particularly limited. Examples of endoscope constituent members include resin members, metal members, and glass members. For example, the endoscope constituent member can be secured to a support member or other member that forms the endoscope by mixing together the individual components to be present in the adhesive according to the present invention, preferably under reduced pressure, injecting or applying the mixture to the area to be bonded, and heating the mixture, for example, at -10°C to 60°C (preferably 0°C to 60°C, more preferably 10°C to 50°C) for 1.5 to 200 hours.

The form of use of the adhesive in the method for manufacturing the endoscope according to the present invention will hereinafter be described with reference to the following specific examples, although these examples are not intended to limit the present invention.

Among endoscope constituent members to be secured with the adhesive according to the present invention, an example of a resin member is a tube for insertion into the insertion section of an endoscope. Examples of resin materials that form the tube include fluorocarbon resins such as Teflon (registered trademark), polysulfones, polyesters, polyolefins, and silicones. For example, the adhesive according to the present invention can be used to bond a metal or glass member that forms the insertion section of an endoscope to the tube (to secure a metal or glass member to the tube).

As described above, the adhesive according to the present invention can also be used to form the adhesive cured product layer 17 in FIG. 2. The adhesive according to the present invention can also be used to bond the resin layer 15 to the coat layer 16 in FIG. 2.

The adhesive according to the present invention can be used for outer surface finishing and securing of an end portion of a flexible outer skin tube (resin layer 15) (an end portion on the distal side (angle portion 3b side) of the flexible tube 3a). Specifically, the end portion of the resin layer 15 of the flexible tube 3a is secured to the inner member by binding the end portion from outside with a thread, and the adhesive is then applied and cured so as to cover the thread. If the adhesive according to the present invention forms the outermost layer of the distal end portion of the flexible tube 3a, the thread on the distal end portion is less likely to come undone, and the insertion section can be more easily inserted into a body cavity.

The adhesive according to the present invention can also be used to bond the tip portion 3c to the angle portion 3b and/or to bond the insertion section 3 to the main-body operating section 5. For example, the tip portion 3c is bonded to the angle portion 3b with the adhesive according to the present invention, the bonded region between the tip portion 3c and the angle portion 3b and the nearby region are bound with a thread to reinforce the bonding, and the adhesive is then applied and cured so as to cover the thread. The insertion section 3 can be similarly bonded to the main-body operating section 5.

The adhesive according to the present invention can also be used to secure various tubes for insertion into the insertion section of an endoscope to the tip portion 3c and/or to the main-body operating section 5.

It is also preferred to use the adhesive according to the present invention to seal the illumination windows 31 and the observation window 32 at the tip portion 3c (to secure glass members). A thick coating of the adhesive smoothens the corner of the rim of a lens and can also block light coming from the side of the lens.

The adhesive according to the present invention can also be used to secure members for purposes such as assembling the imaging device to be built into the tip portion 3c, bonding its parts, and sealing the solid-state imaging element 40. The imaging device has an optical system composed of a plurality of optical elements, such as the lenses L1 to L5 and the prism 38, and the solid-state imaging element 40, such as a charge coupled device (CCD), for photoelectric conversion of an optical image formed by the optical system into image signals. The adhesive according to the present invention can be used, for example, to bond together the optical elements such as the lenses L1 to L5 and the prism 38, which are formed of a material such as glass, and to bond the optical elements such as the lenses L1 to L5 and the prism 38 to the substrate 39, which is formed of resin or metal. By such bonding, glass members can be secured, and metal members can also be secured.

The adhesive according to the present invention can also be used to bond, secure, and seal together the solid-state imaging element 40 and the substrate 39. By such bonding, metal members such as those that form a solid-state imaging element and a substrate can be secured.

Thus, the method for manufacturing an endoscope according to the present invention includes a step of securing an endoscope constituent member with the adhesive according to the present invention.

### Examples

The present invention will now be more specifically described based on the following examples, although these examples should not be construed as limiting the present invention. In the examples below, "room temperature" refers to 25°C. In addition, the amount of a component refers to the amount of the component itself; that is, if the raw material includes a solvent, the amount of the component refers to the amount of the component excluding the solvent.

### Preparation Example: Preparation of Adhesives

The components (a) to (c) listed in the following tables were mixed in the ratios (in parts by mass) listed in the following tables. The resulting mixtures were defoamed at room temperature under a reduced pressure of 1.0 Pa with stirring at 2,000 rpm using "Awatori Rentaro ARV-310 (trade name, manufactured by Thinky Corporation)" for 5 minutes to obtain adhesives. In the following Test Examples, the as-prepared adhesives were used.

### Test Examples

### Fatigue Test (Durability against Mechanical Stress)

Two stainless steel (SUS 304) substrates (with a length of 60 mm, a width of 25 mm, and a thickness of 2 mm) were obtained and placed on top of each other so as to overlap each other by 10 mm in the longitudinal direction (such that the overlapping region had an area of 10 mm × 25 mm). The adhesive was applied to the opposing surfaces over the entire overlapping region and was used to bond the two substrates together. The adhesive layer had a thickness of 0.1 to 0.2 mm. The adhesive was cured by standing at 30°C for 170 hours to obtain a test specimen.

The test specimen was then subjected to vibrations at a repeating load of ±15 kg and a cycle speed of 1,800 cycles/min with a UF-15 universal fatigue testing machine manufactured by Shimadzu Corporation at 23°C and 50% RH. The number of vibrations at which the two bonded SUS substrates became detached from each other was measured and evaluated on the following evaluation scale.

### Evaluation Scale

A: The number of vibrations at which the two SUS substrates became detached was 10,000 or more.
B: The number of vibrations at which the two SUS substrates became detached was 5,000 to less than 10,000.
C: The number of vibrations at which the two SUS substrates became detached was 1,000 to less than 5,000.
D: The number of vibrations at which the two SUS substrates became detached was less than 1,000.

The results are summarized in the following tables.

### Hydrogen Peroxide Plasma Sterilization Durability Test (Durability against Chemical Stress)

Each of the adhesives obtained in the Preparation Example was poured into a Teflon (registered trademark) mold with a length of 100 mm, a width of 20 mm, and a thickness of 0.4 mm and was allowed to stand at 30°C for 170 hours to obtain a sheet-shaped sample (cured product).

The sheet-shaped sample was subjected to hydrogen peroxide plasma sterilization treatment at room temperature using the advanced course of STERRAD (registered trademark) NX (manufactured by Johnson & Johnson). A sheet-shaped sample (I) before sterilization treatment and a sheet-shaped sample (II) repeatedly subjected to hydrogen peroxide plasma sterilization treatment 100 times were subjected as test specimens to a tensile test in which they were pulled at a tensile speed of 20 mm/min and a gauge length of 20 mm in the longitudinal direction using Autograph AGS-X (trade name, manufactured by Shimadzu Corporation).

Breaking strength retention was defined as the proportion of the breaking strength of the sheet-shaped sample (II) to the breaking strength of the sheet-shaped sample (I) (100 × (breaking strength of sheet-shaped sample (II))/(breaking strength of sheet-shaped sample (I))) and was evaluated for sterilization treatment durability on the following evaluation scale.

### Evaluation Scale

AA: The breaking strength retention was 90% or more.
A: The breaking strength retention was 80% to less than 90%.
B: The breaking strength retention was 60% to less than 80%.
C: The breaking strength retention was 40% to less than 60%.
D: The breaking strength was less than 40% of that before the sterilization treatment, or it was impossible to perform the tensile test because the sample was degraded and damaged during the hydrogen peroxide plasma sterilization treatment.

The results are summarized in the following tables.

**Table 1**

| | | | Example 1 | Example 2 | Example 3 | Example 4 | Example 5 | Example 6 | Example 7 | Example 8 | Example 9 (not according to the claimed invention) | Example 10 |
|---|---|---|---|---|---|---|---|---|---|---|---|---|
| Component (a) | Epoxy resin | Type | (a-1) | (a-2) | (a-3) | (a-4) | (a-5) | (a-1) | (a-1) | (a-1) | (a-1) | (a-1) |
| | | Amount | 100 | 100 | 100 | 100 | 100 | 100 | 100 | 100 | 100 | 100 |
| Component (b) | Polyamine | Type | (b-1) | (b-1) | (b-1) | (b-1) | (b-1) | (b-2) | (b-3) | (b-1) | (b-1) | (b-1) |
| | | Amount | 25 | 25 | 25 | 25 | 25 | 25 | 25 | 25 | 25 | 25 |
| Component (c) | Inorganic filler | Type | (c-1) | (c-1) | (c-1) | (c-1) | (c-1) | (c-1) | (c-1) | (c-2) | (c-3) | (c-4) |
| | | Composition | SiO₂ | SiO₂ | SiO₂ | SiO₂ | SiO₂ | SiO₂ | SiO₂ | SiO₂ | SiO₂ | TiO₂ |
| | | Surface treatment | HMDS | HMDS | HMDS | HMDS | HMDS | HMDS | HMDS | DMDS | - | C8 |
| | | Amount | 15 | 15 | 15 | 15 | 15 | 15 | 15 | 15 | 15 | 15 |
| | Amide compound | Type | | | | | | | | | | |
| | | Amount | | | | | | | | | | |
| | Fatty acid wax | Type | | | | | | | | | | |
| | | Amount | | | | | | | | | | |
| | Sorbitol compound | Type | | | | | | | | | | |
| | | Amount | | | | | | | | | | |
| Fatigue test | | | A | A | A | A | A | A | A | A | A | A |
| Sterilization durability | | | AA | AA | AA | A | B | A | A | AA | C | A |

| | | | | | | | | | | | | |
|---|---|---|---|---|---|---|---|---|---|---|---|---|
| HMDS: hexamethyldisilazane C8: octylsilyl | | | | | | | | | | | | |

**Table 2**

| | | | Example 11 | Example 12 | Example 13 | Example 14 (not according to the claimed invention) | Example 15 (not according to the claimed invention) | Example 16 | Example 17 | Example 18 | Example 19 | Example 20 |
|---|---|---|---|---|---|---|---|---|---|---|---|---|
| Component (a) | Epoxy resin | Type | (a-1) | (a-1) | (a-1) | (a-1) | (a-1) | (a-1) | (a-1) | (a-1) | (a-1) | (a-1) |
| | | Amount | 100 | 100 | 100 | 100 | 100 | 100 | 100 | 100 | 100 | 100 |
| Component (b) | Polyamine | Type | (b-1) | (b-1) | (b-1) | (b-1) | (b-1) | (b-1) | (b-1) | (b-1) | (b-1) | (b-1) |
| | | Amount | 25 | 25 | 25 | 25 | 25 | 25 | 25 | 25 | 25 | 25 |
| Component (c) | Inorganic filler | Type | (c-5) | (c-6) | (c-7) | (c-8) | (c-9) | | | | | |
| | | Composition | Al₂O₃ | Bentonite | Talc | CB | CNT | | | | | |
| | | Surface treatment | C8 | TMSA | Silane | - | - | | | | | |
| | | Amount | 15 | 15 | 15 | 15 | 15 | | | | | |
| | Amide compound | Type | | | | | | (c-10) | (c-11) | (c-12) | (c-13) | |
| | | Amount | | | | | | 15 | 15 | 15 | 15 | |
| | Fatty acid wax | Type | | | | | | | | | | (c-14) |
| | | Amount | | | | | | | | | | 15 |
| | Sorbitol compound | Type | | | | | | | | | | |
| | | Amount | | | | | | | | | | |
| Fatigue test | | | A | A | B | C | C | A | A | B | B | A |
| Sterilization durability | | | A | A | A | A | A | A | A | A | A | A |

| | | | | | | | | | | | | |
|---|---|---|---|---|---|---|---|---|---|---|---|---|
| CB: carbon black TMSA: trimethylstearylammonium CNT: carbon nanotube | | | | | | | | | | | | |

**Table 3**

| | | | Example 21 | Example 22 | Example 23 | Example 24 | Example 25 | Example 26 | Example 27 | Example 28 | Example 29 |
|---|---|---|---|---|---|---|---|---|---|---|---|
| Component (a) | Epoxy resin | Type | (a-1) | (a-1) | (a-1) | (a-1) | (a-1) | (a-1) | (a-1) | (a-1) | (a-1) |
| | | Amount | 100 | 100 | 100 | 100 | 100 | 100 | 100 | 100 | 100 |
| Component (b) | Polyamine | Type | (b-1) | (b-1) | (b-1) | (b-1) | (b-1) | (b-1) | (b-1) | (b-1) | (b-1) |
| | | Amount | 25 | 25 | 25 | 25 | 25 | 25 | 25 | 25 | 25 |
| Component (c) | Inorganic filler | Type | | | | | | | (c-1) | (c-1) | (c-1) |
| | | Composition | | | | | | | SiO₂ | SiO₂ | SiO₂ |
| | | Surface treatment | | | | | | | HMDS | HMDS | HMDS |
| | | Amount | | | | | | | 5 | 10 | 20 |
| | Amide compound | Type | | | | | | | | | |
| | | Amount | | | | | | | | | |
| | Fatty acid wax | Type | (c-15) | (c-16) | (c-17) | (c-18) | | | | | |
| | | Amount | 15 | 15 | 15 | 15 | | | | | |
| | Sorbitol compound | Type | | | | | (c-19) | (c-20) | | | |
| | | Amount | | | | | 15 | 15 | | | |
| Fatigue test | | | A | B | B | B | A | A | C | B | A |
| Sterilization durability | | | A | B | B | B | A | A | A | A | A |

**Table 4**

| | | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|---|
| Component (a) | Epoxy resin | Type | (a-1) | (a-2) | (a-3) | (a-4) | (a-5) | (a-1) | (a-1) | (a-1) | (a-1) | (a-1) |
| | | Amount | 100 | 100 | 100 | 100 | 100 | 100 | 100 | 100 | 100 | 100 |
| Component (b) | Polyamine | Type | (b-1) | (b-1) | (b-1) | (b-1) | (b-1) | (b-4) | (b-4) | (b-4) | (b-4) | (b-4) |
| | | Amount | 25 | 25 | 25 | 25 | 25 | 25 | 25 | 25 | 25 | 25 |
| Component (c) | Inorganic filler | Type | | | | | | | (c-1) | | | |
| | | Composition | | | | | | | SiO₂ | | | |
| | | Surface treatment | | | | | | | HMDS | | | |
| | | Amount | | | | | | | 15 | | | |
| | Amide compound | Type | | | | | | | | (c-10) | | |
| | | Amount | | | | | | | | 15 | | |
| | Fatty acid wax | Type | | | | | | | | | (c-14) | |
| | | Amount | | | | | | | | | 15 | |
| | Sorbitol compound | Type | | | | | | | | | | (c-19) |
| | | Amount | | | | | | | | | | 15 |
| Fatigue test | | | D | D | D | D | D | D | A | A | A | A |
| Sterilization durability | | | A | A | A | B | C | D | D | D | D | D |

**Table 5**

| | | | Comparative Example 11 | Comparative Example 12 | Comparative Example 13 | Comparative Example 14 | Comparative Example 15 |
|---|---|---|---|---|---|---|---|
| Component (a) | Epoxy resin | Type | (a-1) | (a-1) | (a-1) | (a-1) | (a-1) |
| | | Amount | 100 | 100 | 100 | 100 | 100 |
| Component (b) | Polyamine | Type | (b-5) | (b-6) | (b-7) | (b-8) | (b-9) |
| | | Amount | 25 | 25 | 25 | 25 | 25 |
| Component (c) | Inorganic filler | Type | (c-1) | (c-1) | (c-1) | (c-1) | (c-1) |
| | | Composition | SiO₂ | SiO₂ | SiO₂ | SiO₂ | SiO₂ |
| | | Surface treatment | HMDS | HMDS | HMDS | HMDS | HMDS |
| | | Amount | 15 | 15 | 15 | 15 | 15 |
| | Amide compound | Type | | | | | |
| | | Amount | | | | | |
| | Fatty acid wax | Type | | | | | |
| | | Amount | | | | | |
| | Sorbitol compound | Type | | | | | |
| | | Amount | | | | | |
| Fatigue test | | | A | A | A | B | C |
| Sterilization durability | | | D | D | D | D | D |

### Component (a): Epoxy Resin

(a-1):
   Bisphenol A diglycidyl ether (trade name "jER 825", manufactured by Mitsubishi Chemical Corporation, epoxy equivalent weight = 170)
(a-2):
   Bisphenol A diglycidyl ether (trade name "jER 828", manufactured by Mitsubishi Chemical Corporation, epoxy equivalent weight = 190)
(a-3):
   Bisphenol A diglycidyl ether (trade name "jER 834", manufactured by Mitsubishi Chemical Corporation, epoxy equivalent weight = 230)
(a-4):
   Bisphenol F diglycidyl ether (trade name "EPICLON 830", manufactured by DIC Corporation, epoxy equivalent weight = 170)
(a-5):
   Epoxy novolac resin (Product No. 406775, manufactured by Sigma-Aldrich Co., epoxy equivalent weight = 170)

### Component (b): Polyamine

(b-1):
   Polyoxyalkylenetriamine (trade name: T403, manufactured by Mitsui Fine Chemicals, Inc, active hydrogen equivalent weight = 73)
(b-2):
   Polyoxyalkylenediamine (trade name: D2000, manufactured by Mitsui Fine Chemicals, Inc, active hydrogen equivalent weight = 500)
(b-3):
   Polyoxyalkylenediamine (trade name: D400, manufactured by Mitsui Fine Chemicals, Inc, active hydrogen equivalent weight = 100)
(b-4):
   Polyamide-amine (trade name: ST13, manufactured by Mitsubishi Chemical Corporation)
(b-5):
   m-Xylenediamine (trade name: MXDA, manufactured by Mitsubishi Gas Chemical Company, Inc.)
(b-6):
   Tetraethylenepentamine (manufactured by Tokyo Chemical Industry Co., Ltd.)
(b-7):
   1,3-Bisaminomethylcyclohexane (trade name: 1,3-BAC, manufactured by Mitsubishi Gas Chemical Company, Inc.)
(b-8):
   Isophoronediamine (manufactured by Tokyo Chemical Industry Co., Ltd.)
(b-9):
   m-Phenylenediamine (manufactured by Tokyo Chemical Industry Co., Ltd.)

### Component (c): Inorganic Filler, Amide Compound, Fatty Acid Wax, Sorbitol Compound

### Inorganic Filler

(c-1):
   Hexamethyldisilazane surface-treated silica (trade name: AEROSIL RX200, primary particle size = 12 nm, manufactured by Nippon Aerosil Co., Ltd.)
(c-2):
   Dimethyldichlorosilane surface-treated silica (trade name: AEROSIL R9200, primary particle size = 12 nm, manufactured by Nippon Aerosil Co., Ltd.)
(c-3):
   Untreated silica (trade name: AEROSIL 200, primary particle size = 12 nm, manufactured by Nippon Aerosil Co., Ltd.)
(c-4):
   Octylsilyl surface-treated titanium oxide (trade name: AEROXIDE TiO₂ NKT90, primary particle size = 14 nm, manufactured by Nippon Aerosil Co., Ltd.)
(c-5):
   Octylsilyl surface-treated alumina (trade name: AEROXIDE Alu C805, primary particle size = 13 nm, manufactured by Nippon Aerosil Co., Ltd.)
(c-6):
   Trimethylstearylammonium-treated bentonite (trade name: KUNIBIS-110, manufactured by Kunimine Industries Co., Ltd.)
(c-7):
   Silane-treated talc (trade name: FH104S, average particle size = 4 µm, manufactured by Fuji Talc Industrial Co., Ltd.)
(c-8):
   Carbon black (trade name: #1000, manufactured by Mitsubishi Chemical Corporation)
(c-9):
   Multi-walled carbon nanotube (trade name: MWNT MTC, manufactured by Meijo Nano Carbon Co., Ltd.)

### Amide Compound

(c-10):
   Ethylene bisstearamide (trade name: SLIPACKS E, manufactured by Mitsubishi Chemical Corporation)
(c-11):
   Hexamethylene bishydroxystearamide (trade name: SLIPACKS ZHH, manufactured by Mitsubishi Chemical Corporation)
(c-12):
   Stearamide (trade name: NEUTRON-2, manufactured by Nippon Fine Chemical Co., Ltd.)
(c-13):
   N-Oleylpalmitamide (trade name: PNT-34, manufactured by Nippon Fine Chemical Co., Ltd.)

### Fatty Acid Wax

(c-14):
   Hydrogenated castor oil (trade name: DISPARLON 308, manufactured by Kusumoto Chemicals, Ltd.)
(c-15):
   Polyoxyethylene hardened castor oil (trade name: EMANON CH-25, manufactured by Kao Corporation)
(c-16):
   Stearyl stearate (trade name: EXCEPARL SS, manufactured by Kao Corporation)
(c-17):
   1-Triacontanol (manufactured by FUJIFILM Wako Pure Chemical Corporation)
(c-18):
   1-Triacontanoic acid (manufactured by Tokyo Chemical Industry Co., Ltd.)

### Sorbitol Compound

(c-19):
   1,3:2,4-bis-O-Benzylidene-D-glucitol (also known as 1,3:2,4-dibenzylidenesorbitol) (trade name: GELALL D, manufactured by New Japan Chemical Co., Ltd.)
(c-20):
   1,3:2,4-bis-O-(4-Methylbenzylidene)-D-sorbitol (also known as 1,3:2,4-bis(p-methylbenzylidene)sorbitol) (trade name: GELALL MD, manufactured by New Japan Chemical Co., Ltd.)

As shown in the tables, the cured products obtained from the adhesives in which an epoxy resin serving as the component (a) and a polyamine compound serving as the component (b) were used in combination as adhesive components but which contained no component (c) exhibited low durability against mechanical stress (Comparative Examples 1 to 5). In addition, the cured products obtained from the adhesives in which the structure of the polyamine compound did not satisfy the requirement regarding the component (b) exhibited low durability against chemical sterilization treatment (Comparative Examples 6 to 15).

In contrast, the adhesives containing all components (a) to (c) exhibited less degradation after repeated exposure to mechanical stress or after repeated exposure to chemical sterilization treatment (Examples 1 to 29).

This application claims priority from JP2019-032973 filed in Japan on February 26, 2019.

### Reference Signs List

- 2: electronic endoscope (endoscope)
- 3: insertion section
- 3a: flexible tube
- 3b: angle portion
- 3c: tip portion
- 5: main-body operating section
- 6: universal cord
- 11: spiral tube
- 11a: metal strip
- 12: tubular net
- 13: cap
- 14: flexible tube substrate
- 14a: distal side
- 14b: proximal side
- 15: resin layer
- 16: coat layer
- 17: adhesive cured product layer
- 31: illumination window
- 32: observation window
- 33: forceps port
- 34: nozzle
- 35: tip-portion main body
- 36: distal end cap
- 37: lens holder
- 38: prism
- 39: substrate
- 40: solid-state imaging element
- 41: adhesive cured product
- 42: adhesive cured product
- 43: observation unit
- L1 to L5: lens
- 43: observation unit
- L1 to L5: lens

## Claims

1. An adhesive for an endoscope, comprising the following (a) to (c):
(a) an epoxy resin including at least one of a bisphenol A epoxy resin, a bisphenol F epoxy resin, or a phenol novolac epoxy resin;
(b) a polyamine compound having an oxygen atom but no amide bond in a molecule thereof; and
(c) at least one of an alkyl-modified inorganic filler, an amide compound, a fatty acid wax, or a sorbitol compound;
wherein the alkyl-modified inorganic filler has at least an alkyl group introduced onto a surface of an inorganic filler by a surface treatment.

2. The adhesive for an endoscope according to claim 1, wherein the polyamine compound has an oxyalkylene structure.

3. The adhesive for an endoscope according to claim 1 or 2, wherein the inorganic filler is at least one of silica, titanium oxide, aluminum oxide, or a layered silicate.

4. The adhesive for an endoscope according to any one of claims 1 to 3, wherein the amide compound is a reaction product of a fatty acid with a polyamine.

5. The adhesive for an endoscope according to any one of claims 1 to 4, wherein the fatty acid wax is hardened castor oil.

6. The adhesive for an endoscope according to any one of claims 1 to 5, wherein the sorbitol compound is at least one of a dibenzylidene sorbitol compound or a tribenzylidene sorbitol compound.

7. The adhesive for an endoscope according to any one of claims 1 to 6, wherein the amount of the polyamine compound is 15 to 50 parts by mass, based on 100 parts by mass of the epoxy resin.

8. The adhesive for an endoscope according to any one of claims 1 to 7, wherein the amount of component (c) present in the adhesive is from 1 to 50 parts by mass, based on 100 parts by mass of the epoxy resin.

9. A cured product obtained by curing the adhesive for an endoscope according to any one of claims 1 to 8.

10. An endoscope comprising a constituent member secured with the cured product according to claim 9.

11. A method for manufacturing an endoscope, comprising securing a constituent member with the adhesive for an endoscope according to any one of claims 1 to 8.

## Patentansprüche

1. Klebstoff für ein Endoskop, umfassend die folgenden (a) bis (c):
(a) ein Epoxidharz, das mindestens eines von einem Bisphenol-A-Epoxidharz, einem Bisphenol-F-Epoxidharz und einem Phenol-Novolac-Epoxidharz enthält;
(b) eine Polyaminverbindung, die ein Sauerstoffatom, aber keine Amidbindung in einem Molekül davon aufweist; und
(c) mindestens eines von einem alkylmodifizierten anorganischen Füllstoff, einer Amidverbindung, einem Fettsäurewachs und einer Sorbitolverbindung;
wobei der alkylmodifizierte anorganische Füllstoff mindestens eine Alkylgruppe aufweist, die durch eine Oberflächenbehandlung auf eine Oberfläche eines anorganischen Füllstoffs eingeführt wurde.

2. Klebstoff für ein Endoskop nach Anspruch 1, wobei die Polyaminverbindung eine Oxyalkylenstruktur aufweist.

3. Klebstoff für ein Endoskop nach Anspruch 1 oder 2, wobei der anorganische Füllstoff mindestens eines von Siliciumdioxid, Titanoxid, Aluminiumoxid und einem Schichtsilikat ist.

4. Klebstoff für ein Endoskop nach einem der Ansprüche 1 bis 3, wobei die Amidverbindung ein Reaktionsprodukt einer Fettsäure mit einem Polyamin ist.

5. Klebstoff für ein Endoskop nach einem der Ansprüche 1 bis 4, wobei das Fettsäurewachs gehärtetes Rizinusöl ist.

6. Klebstoff für ein Endoskop nach einem der Ansprüche 1 bis 5, wobei die Sorbitolverbindung mindestens eines von einer Dibenzyliden-Sorbitolverbindung und einer Tribenzyliden-Sorbitolverbindung ist.

7. Klebstoff für ein Endoskop nach einem der Ansprüche 1 bis 6, wobei die Menge der Polyaminverbindung 15 bis 50 Massenteile beträgt, basierend auf 100 Massenteilen des Epoxidharzes.

8. Klebstoff für ein Endoskop nach einem der Ansprüche 1 bis 7, wobei die Menge an Komponente (c), die in dem Klebstoff vorhanden ist, 1 bis 50 Massenteile beträgt, basierend auf 100 Massenteilen des Epoxidharzes.

9. Gehärtetes Produkt, das durch Härtung des Klebstoffs für ein Endoskop nach einem der Ansprüche 1 bis 8 erhalten wird.

10. Endoskop, umfassend einen Bestandteil, der mit dem gehärteten Produkt nach Anspruch 9 gesichert ist.

11. Verfahren zum Herstellen eines Endoskops, umfassend Sichern eines Bestandteils mit dem Klebstoff für ein Endoskop nach einem der Ansprüche 1 bis 8.

## Revendications

1. Adhésif pour un endoscope, comprenant les éléments suivants (a) à (c) :
(a) une résine époxy incluant au moins l'une d'une résine époxy de bisphénol A, d'une résine époxy de bisphénol F ou d'une résine époxy phénol novolaque ;
(b) un composé de polyamine ayant un atome d'oxygène mais aucune liaison amide dans une molécule de celui-ci ; et
(c) au moins l'un d'une charge inorganique modifiée par alkyle, d'un composé amide, d'une cire d'acide gras ou d'un composé de sorbitol ;
dans lequel la charge inorganique modifiée par alkyle comporte au moins un groupe alkyle introduit sur une surface d'une charge inorganique par un traitement de surface.

2. Adhésif pour un endoscope selon la revendication 1, dans lequel le composé de polyamine présente une structure oxyalkylène.

3. Adhésif pour un endoscope selon la revendication 1 ou la revendication 2, dans lequel la charge inorganique est au moins l'une de la silice, de l'oxyde de titane, de l'oxyde d'aluminium ou d'un silicate lamellaire.

4. Adhésif pour un endoscope selon l'une quelconque des revendications 1 à 3, dans lequel le composé amide est un produit de réaction d'un acide gras avec une polyamine.

5. Adhésif pour un endoscope selon l'une quelconque des revendications 1 à 4, dans lequel la cire d'acide gras est de l'huile de ricin durcie.

6. Adhésif pour un endoscope selon l'une quelconque des revendications 1 à 5, dans lequel le composé de sorbitol est au moins l'un d'un composé de dibenzylidène sorbitol ou d'un composé de tribenzylidène sorbitol.

7. Adhésif pour un endoscope selon l'une quelconque des revendications 1 à 6, dans lequel la quantité du composé de polyamine est de 15 à 50 parties en masse, sur la base de 100 parties en masse de la résine époxy.

8. Adhésif pour un endoscope selon l'une quelconque des revendications 1 à 7, dans lequel la quantité du composant (c) présent dans l'adhésif est de 1 à 50 parties en masse, sur la base de 100 parties en masse de la résine époxy.

9. Produit durci obtenu par durcissement de l'adhésif pour un endoscope selon l'une quelconque des revendications 1 à 8.

10. Endoscope comprenant un élément constitutif fixé avec le produit durci selon la revendication 9.

11. Procédé de fabrication d'un endoscope, comprenant fixer un élément constitutif avec l'adhésif pour un endoscope selon l'une quelconque des revendications 1 à 8.
